(19) **Europäisches Patentamt European Patent Office Office européen des brevets**

(11) **EP 1 776 082 B1**

(12) **FASCICULE DE BREVET EUROPEEN**

(45) Date de publication et mention de la délivrance du brevet:
**21.01.2009 Bulletin 2009/04**

(21) Numéro de dépôt: **05760048.8**

(22) Date de dépôt: **21.04.2005**

(51) Int Cl.:
*A61K 8/97* (2006.01)     *A61K 8/64* (2006.01)
*A61K 36/31* (2006.01)     *A61Q 19/02* (2006.01)
*A61Q 5/08* (2006.01)

(86) Numéro de dépôt international:
**PCT/FR2005/000985**

(87) Numéro de publication internationale:
**WO 2005/107697 (17.11.2005 Gazette 2005/46)**

(54) **UTILISATION D'UN HYDROLYSAT DE PROTEINES DE CRUCIFERES EN TANT QU'AGENT DEPIGMENTANT DANS OU POUR UNE COMPOSITION COSMETIQUE ET/OU PHARMACEUTIQUE**

VERWENDUNG EINES KREUZBLÜTLER-PROTEINHYDROLYSATS ALS DEPIGMENTIERUNGSMITTEL ODER FÜR EINE KOSMETISCHE UND/ODER PHARMAZEUTISCHE ZUSAMMENSETZUNG

USE OF A CRUCIFEROUS PROTEIN HYDROLYSATE AS A DEPIGMENTATION AGENT OR FOR A COSMETIC AND/OR PHARMACEUTICAL COMPOSITION

(84) Etats contractants désignés:
**AT BE BG CH CY CZ DE DK EE ES FI FR GB GR HU IE IS IT LI LT LU MC NL PL PT RO SE SI SK TR**

(30) Priorité: **21.04.2004 FR 0404192**

(43) Date de publication de la demande:
**25.04.2007 Bulletin 2007/17**

(73) Titulaire: **SOCIETE D'EXTRACTION DES PRINCIPES ACTIFS SA (VINCIENCE) 06904 Sofia Antipolis (FR)**

(72) Inventeurs:
• **DAL FARRA, Claude F-06650 Opio (FR)**
• **DOMLOGE, Nouha F-06560 Valbonne (FR)**
• **PEYRONEL, Dominique F-13014 Marseille (FR)**

(74) Mandataire: **Macquet, Christophe Macquet & Associés Arche des Dolines 7, rue Soutrane 06560 Sophia Antipolis (FR)**

(56) Documents cités:
**EP-A- 1 410 721        WO-A-01/28353
WO-A-20/04057976        DE-A- 19 521 165
FR-A- 2 676 741        US-A- 4 959 350**

• **DATABASE BIOSIS [Online] BIOSCIENCES INFORMATION SERVICE, PHILADELPHIA, PA, US; avril 2000 (2000-04), VIOQUE JAVIER ET AL: "Partially hydrolyzed rapeseed protein isolates with improved functional properties" XP002306696 Database accession no. PREV200000240391 & JOURNAL OF THE AMERICAN OIL CHEMISTS' SOCIETY, vol. 77, no. 4, avril 2000 (2000-04), pages 447-450, ISSN: 0003-021X**

**Description**

L'invention concerne le domaine de la cosmétique et de la pharmaceutique, notamment le domaine de la dermatologie. La présente invention a pour objet l'utilisation d'une quantité efficace d'un hydrolysat de protéines de plantes appartenant à la famille des crucifères, en tant qu'agent blanchissant et/ou dépigmentant de la peau, pour la préparation d'une composition thérapeutique pour blanchir et/ou dépigmenter, ainsi que l'utitlisation non-thérapeutique de cet hydrolysat pour blanchir et/ou dépigmenter

La couleur de la peau et des cheveux des mammifères est sous l'influence de différentes facteurs, des facteurs génétiques, bien sûr, mais aussi environnementaux tels que, par exemple, l'exposition au soleil. Cette couleur repose essentiellement sur la présence d'un pigment particulier, la mélanine. Cette mélanine joue en effet un rôle fondamental dans la détermination de la couleur de la peau. Elle est synthétisée par de larges cellules dendritiques : les mélanocytes, cellules localisées dans la jonction dermo-épidermique. La mélanine existe sous deux formes différentes : la phaeomélanine, pigment de couleur jaune, et l'eumélanine de couleur noire. Ce sont les différentes proportions et la taille de ces pigments, sans oublier les caroténoïdes et la micro-circulation sanguine, qui donnent à la peau sa grande diversité de couleur.

Les deux types de mélanines sont synthétisés à partir d'un même acide aminé, la tyrosine. Cette synthèse est sous la dépendance d'une enzyme clé, la tyrosinase, qui transforme la tyrosine en DOPA, puis en DOPA-quinone. La DOPA-quinone donnant naissance à la phaeomélanine ou bien à l'eumélanine. En présence de cystéine, un acide aminé riche en soufre, la DOPA-quinone sera transformée en Cystéinyl-DOPA, intermédiaire dans la synthèse de phaeomélanine. Tandis qu'en l'absence de cystéine, de l'indole 5-6 quinone sera formé et de l'eumélanine sera synthétisée. Les mélanocytes transfèrent ensuite la mélanine dans les cellules adjacentes, c'est-à-dire les kératinocytes.

La production de mélanine, ainsi que son transport, sont régulés par différents facteurs tels que, par exemple, le rayonnement UV, les hormones ou les produits chimiques. Ainsi, une augmentation de l'exposition aux rayonnements UV provoque la synthèse de pigments et résulte en un assombrissement de la peau. Des perturbations de cette pigmentation, plus ou moins bénignes, peuvent apparaître. Elles se manifestent, par exemple, par des taches de rousseur, des grains de beauté, des taches diffuses telles que les taches de grossesse, le chloasma ainsi que par d'autres désordres hyperpigmentaires comme par exemple le lentigo. En outre, le vieillissement peut moduler la pigmentation cutanée. Ainsi, certaines personnes peuvent voir apparaître des taches sur la peau, plus ou moins foncées ou colorées, conférant des zones de colorations hétérogènes formant des taches de sénescence.

L'utilisation d'inhibiteur ou de régulateur de la synthèse de mélanine, ainsi que de tout autre produit dépigmentant et/ou blanchissant, est donc particulièrement intéressant en cosmétologie et/ou en dermatologie. Cette utilisation est non seulement intéressante lorsqu'une véritable dépigmentation de la peau est recherchée, comme dans le cas de blanchiment de peau fortement pigmentée ou encore lors d'inhibition de zones cutanées hyperpigmentées résultant en un aspect inesthétique de la peau ; mais elle l'est également dans certaines applications visant à éclaircir le teint, à donner de la luminosité à la peau ou encore à donner de l'éclat aux tissus de surface.

Jusqu'à ce jour, de nombreuses molécules ont été proposées mais très peu sont véritablement utilisées car beaucoup d'entre elles présentent des problèmes d'irritation voire de toxicité. Parmi ces molécules, on peut citer des dérivés du phénol tels que l'hydroquinone et le résorcinol qui inhibent une série de réaction de la conversion de la L-tyrosine en mélanine par inhibition de l'activité de la tyrosinase (Takano, 1984). On peut citer aussi l'acide L-ascorbique et ses dérivés, le magnésium ascorbyl acétate, l'acide kojique ou encore l'acide lactique. De nouveaux produits et molécules ont été mis au point afin de résoudre ces problèmes. Ainsi, le document GB 1349955 décrit une composition blanchissante contenant de l'hydroquinone, un agent desquamant et un corticostéroïde anti-inflammatoire. Le document EP98401360 décrit l'utilisation de sulfites ou métalsulfites dans une composition cosmétique à activité dépigmentante.

Un certain nombre de substances introduites dans des produits cosmétiques ou pharmaceutiques ont ainsi vu le jour. Mais il reste, cependant, encore des progrès à faire afin de pouvoir disposer de produits capables de régler ces problèmes de manière satisfaisante. Il subsiste, notamment, le besoin d'une composition dépigmentante et/ou blanchissante qui, bien qu'étant convenablement tolérée par la peau, soit plus efficace que les compositions mentionnées ci-dessus.

Le problème technique à résoudre a donc été, pour les inventeurs, de trouver une nouvelle substance, cosmétiquement ou pharmaceutiquement acceptable, qui soit capable d'avoir une réelle activité blanchissante et/ou dépigmentante au niveau de la peau tout en n'ayant pas des effets secondaires indésirables tels que, par exemple, des réactions de toxicité ou d'irritation cutanée.

Les inventeurs ont réussi à sélectionner des substances particulières présentant des propriétés remarquables lorsque celles-ci sont appliquées sur la peau. Ils ont, notamment, découvert qu'un hydrolysat de protéines de plantes appartenant à la famille des crucifères a des propriétés remarquables sur la peau et, plus particulièrement, des propriétés blanchissantes. Ce composé permet, en effet, d'inhiber significativement la synthèse de mélanine des cellules de la peau.

Les végétaux de la famille des crucifères (latinisée en *Cruciferae*) se regroupent dans une large famille qui compte environ 3200 espèces réparties en 375 genres à travers le monde. Les crucifères sont également appelées *Brassicaceae.* Ces plantes se trouvent principalement dans les régions tempérées de l'hémisphère Nord et, plus particulièrement, dans le pourtour méditerranéen. Les crucifères tirent leur nom de la disposition en croix de leurs sépales et pétales. Ce sont des plantes herbacées, vivaces, annuelles et assez couramment bisannuelles. Leur racine est pivotante et tubérisée. Le feuillage est alterne, à stipules réduites et caduques, voire même absentes. Le fruit est une silique, sa forme, sa longueur ou encore son épaisseur servent à la reconnaissance des espèces. Les graines se détachent progressivement ; elles sont dépourvues d'albumen, les réserves étant essentiellement d'origine lipidique. Comme plante appartenant à cette famille on peut citer des plantes de grandes cultures, oléagineuses, tel le colza (Brassica napus, var. oleifera) ; des plantes alimentaires comme toutes les variétés de "chou" (du genre Brassica) ; des condiments tel que la moutarde (du genre Sinapis) ; ou encore des plantes à intérêt ornemental tel que la giroflée ou la corbeille d'or (du genre Alyssum).

Il a déjà été proposé, dans des documents antérieurs, des compositions cosmétiques contenant des extraits de Brassicaceae. Ainsi, par exemple, le brevet FR 2802417 décrit une préparation cosmétique et/ou pharmaceutique contenant une quantité efficace d'un extrait de Brassicaceae et un corps gras et/ou des émulsifiants. Cependant, à la connaissance de la demanderesse, il n'a jamais été décrit, dans l'art antérieur, l'utilisation d'un hydrolysat de protéines de plantes appartenant à la famille des crucifères, en tant qu'agent blanchissant et/ou dépigmentant en cosmétique et/ou en dermatologie et/ou en pharmaceutique.

Ainsi, selon un premier aspect, la présente invention a pour objet l'utilisation d'une quantité efficace d'un hydrolysat de protéines de plantes appartenant à la famille des crucifères comme principe actif blanchissant pour la préparation d'une composition thérapeutique pour blanchir et/ou dépigmenter, ainsi que son utilisation non-thérapeutique pour blanchir et/ou dépigmenter. Selon un mode de réalisation de l'invention tout particulièrement préféré, l'hydrolysat est un hydrolysat de protéines fermentées de plantes appartenant à la famille des crucifères.

Le terme "hydrolysat" désigne toute substance ayant subi une hydrolyse. L'hydrolyse se définit comme le clivage d'une molécule par une molécule d'eau. L'hydrolyse peut être enzymatique ou chimique, préférentiellement selon l'invention l'hydrolyse est enzymatique. Un hydrolysat de protéines désigne donc le produit obtenu après hydrolyse des protéines de la plantes. L'hydrolyse des protéines, plus ou moins poussée, permet d'obtenir un hydrolysat contenant soit des peptides de poids moléculaires variables, soit des acides aminés. Les protéines ainsi hydrolysées ont été examinées pour leurs propriétés dans le domaine des cosmétiques et de la dermatologie.

Préférentiellement selon l'invention, l'hydrolysat de protéines est préparé à partir de protéines fermentées, c'est-à-dire à partir de protéines qui ont subi une étape de fermentation. Par fermentation, on entend une transformation des substances organiques sous l'action de micro-organismes. Préférentiellement selon l'invention, les micro-organismes utilisés sont des levures, et plus particulièrement des levures du genre Rhizopus, Aspergillus ou Pénicillium.

L'hydrolysat de protéines de plantes appartenant à la famille des crucifères doit s'entendre comme un hydrolysat d'au moins un végétal appartenant à la famille des crucifères. Bien entendu cet hydrolysat peut être préparé à partir d'au moins l'un quelconque des nombreux genres et espèces appartenant à la famille des crucifères. Selon un mode de réalisation avantageux de l'invention, la plante utilisée afin d'obtenir l'hydrolysat de protéines fermentées, appartenant à la famille des crucifères, est le colza (Brassica napus). Préférentiellement, l'hydrolysat de protéines de plantes appartenant à la famille des crucifères est obtenu à partir de la graine de ces végétaux. Ainsi, selon une méthode de réalisation préférée de l'invention, l'hydrolysat de protéines est obtenu à partir des graines de colza.

Toute méthode d'extraction ou de purification connue de l'homme du métier peut être utilisée afin de préparer l'hydrolysat selon l'invention. On peut, par exemple, dans une première étape, délipider des graines de crucifères, telles que des graines de colza, par un simple pressage et/ou par l'action d'un solvant organique classique (comme par exemple un alcool, un hexane ou de l'acétone). Après séchage du produit ainsi obtenu, on obtient un résidu enrichi en protéines communément appelé « tourteau ». Une étape de fermentation est, ensuite, avantageusement réalisée à partir de ce tourteau.

Selon une autre technique, on peut envisager de réaliser l'extraction d'une fraction protéique obtenue à partir de ce tourteau, cette fraction protéique servira ensuite de substrat à la fermentation. L'étape d'extraction des protéines proprement dite est réalisée en milieu aqueux, neutre ou basique. Préférentiellement selon l'invention l'extraction des protéines sera réalisée en milieu aqueux, légèrement basique, et à chaud. Les protéines seront récupérées par précipitation ou par concentration.

L'étape de fermentation est réalisée de préférence avec des levures, préférentiellement avec des levures des genres Rhizopus, Aspergillus ou Pénicillium. Dans le milieu de fermentation, l'extrait de colza, source de matières azotées, est supplémenté en sucres (glucoses) ainsi qu'en divers éléments nécessaires à la croissance des levures, tels que, par exemple, des acides aminés et des sels minéraux. Une faible concentration d'alcool ou de glycérol peut être ajoutée. La culture s'effectue en fermenteur sous agitation lente (10 à 60 tours/min), à une température comprise entre 20 et 40°C et à un pH variant de 5 à 7,5, le débit d'air étant constant. La durée de cette étape est très variable, elle peut en effet varier de douze heures à vingt jours. Le milieu de culture est, par la suite, soumis à un traitement thermique, c'est-à-dire à une température comprise entre 80°C et 135°C.

L'étape finale d'hydrolyse des protéines est réalisée par des protéases d'origine végétale telle que, par exemple, la papaïne ou bromélaïne ; ou bien par des enzymes dites « industrielles » telles que les alcalases, la flavourzyme... Le milieu de culture ainsi hydrolysé est centrifugé, filtré jusqu'à l'obtention d'un hydrolysat de protéines fermentées de crucifères.

Cet hydrolysat est mis en solution dans un ou plusieurs solvants. On peut en particulier citer des solvants aqueux. Par solvant aqueux, on entend tout solvant constitué totalement ou partiellement d'eau. On peut citer l'eau elle-même, les solvants hydroalcooliques en toutes proportions ou encore les solvants constitués d'eau et d'un composé comme le propylène glycol ou le butylène glycol en toutes proportions.

Les hydrolysats de protéines fermentées de plantes appartenant à la famille des crucifères sont analysés pour leur teneur en composés de nature protéique. On entend, par composés de nature protéique, les fragments de protéines, les peptides et les acides aminés libres présents dans le mélange. Les peptides, acides aminés et fragments de protéines sont dosés selon les techniques classiques, bien connues de l'homme du métier. Ainsi, selon un mode de réalisation avantageux de l'invention, l'hydrolysat contient une quantité de composés de nature protéique représentant entre 30 et 90 % du poids total de la matière sèche, plus particulièrement cette quantité est comprise entre 50 et 80 % du poids total de la matière sèche.

Préférentiellement selon l'invention l'hydrolysat de protéines de plantes appartenant à la famille des crucifères est un hydrolysat de protéines fermentées, c'est-à-dire ayant subi une étape de fermentation.

L'agent actif selon l'invention, ou la composition le contenant, permettra d'éclaircir, voire de blanchir la peau. La peau pouvant être, au départ, plus ou moins colorée ou plus ou moins foncée ; cette couleur ayant une origine naturelle, ou bien étant sous l'influence de facteurs extérieurs tel que, par exemple, le rayonnement UV ou l'âge. Par ailleurs, l'agent actif selon l'invention, ou la composition le contenant, permettra, d'une manière plus ou moins directe, de faire disparaître les taches pigmentaires de la peau et/ou permettra de dépigmenter les poils et/ou les cheveux. Il permettra ainsi de faire éclaircir les régions hyperpigmentées, c'est-à-dire les zones cutanées contenant une grande quantité de mélanine. Par taches pigmentaires de la peau, on entend toutes les modifications de la pigmentation de la peau résultant en un assombrissement général ou bien en un assombrissement localisé, formant ainsi des taches plus ou moins sombres. Ces modifications peuvent être d'origines naturelles ou induites par différents agents comme, par exemple, le rayonnement UV ou encore les produits chimiques. Ces désordres d'ordres pigmentaires peuvent se manifester par des taches de rousseurs des grains de beauté ou des taches diffuses telles que les taches de grossesses, le chloasma ainsi que par d'autres désordres hyperpigmentaires comme par exemple le lentigo. Des perturbations de cette pigmentation, plus ou moins bénignes, peuvent aussi apparaître naturellement avec le vieillissement. Certaines personnes peuvent ainsi voir apparaître des taches sur la peau plus ou moins foncées et/ou colorées, conférant à la peau des zones de colorations hétérogènes formant des taches de sénescence. Plus généralement, l'hydrolysat selon l'invention permet de réguler la pigmentation cutanée.

L'actif selon l'invention est un agent actif blanchissant ou dépigmentant efficace qui agit, entre autres, en inhibant la formation de mélanine au niveau des mélanocytes. Ainsi, selon un autre aspect, l'invention est relative à l'utilisation d'un hydrolysat de protéines fermentées de plantes appartenant à la famille des crucifères, tel que défini précédemment, afin d'inhiber et/ou de diminuer l'activité de la tyrosinase, et/ou afin d'inhiber et/ou de diminuer la synthèse de mélanine.

L'invention concerne l'utilisation d'une composition cosmétique et/ou la préparation d'une composition dermatologique contenant un actif dépigmentant afin d'obtenir un éclaircissement de la peau ou afin de traiter les taches pigmentaires. La composition selon l'invention peut être une composition cosmétique et/ou dermatologique et/ou pharmaceutique. Préférentiellement selon l'invention, la composition est une composition cosmétique, car elle est destinée à améliorer l'aspect et les performances cutanées générales de l'individu qui en fait usage. Plus particulièrement, cette composition est adaptée à une utilisation dans le but de blanchir et/ou de décolorer la peau, de dépigmenter les poils et/ou les cheveux, ou de traiter les taches pigmentaires de la peau. La composition selon l'invention est préférentiellement une composition cosmétique et/ou dermatologique adaptée à l'administration par voie topique cutanée comprenant un milieu cosmétiquement ou dermatologiquement acceptable. Il est bien évident que l'invention s'adresse aux mammifères en général et plus particulièrement aux êtres humains.

La quantité efficace de principe actif correspond à la quantité nécessaire afin d'obtenir le résultat désiré. Selon un mode de réalisation avantageux de l'invention, l'hydrolysat de protéines précité est présent, dans les compositions de l'invention, à une concentration comprise entre 0,0001 % et 20 % environ, et préférentiellement à une concentration comprise entre 0,01 % et 10 % environ en poids par rapport au poids total de la composition finale.

Selon un mode de réalisation avantageux de l'invention, l'hydrolysat précité est préalablement solubilisé dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés ou tout mélange de ces solvants. Selon encore un autre mode de réalisation avantageux de l'invention, l'hydrolysat précité est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans ou fixés sur, tout vecteur cosmétiquement ou pharmaceutiquement acceptable.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisés. Préférentiellement, les compositions selon la présente invention se présenteront sous une forme galénique adaptée à l'administration par voie topique cutanée. Elles couvrent toutes les formes cosmétiques ou dermatologiques. Ces compositions doivent donc contenir un milieu cosmétiquement acceptable, c'est-à-dire compatible avec la peau, les poils ou les cheveux.

Ces compositions peuvent notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions ou encore de poudres, adaptées à une application sur la peau, les muqueuses, les lèvres et/ou les cheveux. Ces compositions peuvent être plus ou moins fluides et avoir l'aspect d'une crème, d'une lotion, d'un lait, d'un sérum, d'une pommade, d'un shampooing, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif usuellement utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc. Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition. Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme composition cosmétique ou pharmaceutique pour la peau, mais aussi comme composition cosmétique pour les cheveux et/ou les poils. Elles trouvent une application toute particulière en tant que produit de dépigmentation et/ou de décoloration de la peau et/ou des poils. La composition mise en oeuvre selon l'invention peut aussi être une composition permettant de lutter contre les taches pigmentaires de la peau.

Selon l'invention, on peut ajouter, entre autres, à la composition divers agents actifs destinés, notamment, à la prévention et/ou au traitement des désordres d'ordre pigmentaire. Par ailleurs, la composition selon l'invention peut associer, à l'hydrolysat de protéines tel que défini précédemment, d'autres agents actifs favorisant son action. Ainsi, il peut être ajouté des agents actifs ayant une action kératolytique, c'est-à-dire des agents desquamants ayant une action exfoliante, tels que, par exemples, les alpha-hydroxyacides et les béta-hydroxyacides. Ces agents agissant de manière efficace sur les mécanismes de la pigmentation.

Selon un autre aspect, la composition selon l'invention peut être une composition solaire, c'est-à-dire une composition aidant à la protection contre le rayonnement solaire. Ainsi, il peut être avantageusement ajouté, à la composition selon l'invention, des actifs aidant à la protection solaire tel que, par exemple, des filtres solaires.

Les compositions, objet de l'invention, trouvent leur application dans grand nombre de traitements notamment cosmétiques ou dermatologiques. Elles peuvent constituer une composition cosmétique, notamment pour le traitement, la protection, le soin, le démaquillage et/ou le nettoyage de la peau et/ou des cheveux, et/ou pour le maquillage de la peau, des lèvres, des cils et/ou du corps. La composition selon l'invention peut également consister en des préparations solides comprenant également des savons ou des pains de nettoyage. La composition peut être aussi conditionnée sous forme d'une composition pour aérosol comprenant également un agent propulseur sous pression.

Selon un autre aspect, la présente invention concerne un procédé de traitement cosmétique destiné à éclaircir la peau et/ou les poils. L'invention concerne aussi un procédé de traitement cosmétique destiné à traiter les désordres de la pigmentation de la peau. Ces procédés, de traitement cosmétique de la peau et/ou des cheveux, consistent à appliquer sur la surface de la peau, les poils ou les cheveux, une quantité efficace d'un hydrolysat de protéines de plantes de la famille des crucifères tel que défini précédemment, afin d'obtenir l'action désirée. Préférentiellement, le procédé consiste à appliquer sur la surface de la peau, les poils ou les cheveux, une quantité efficace d'un hydrolysat de protéines fermentées de plantes de la famille des crucifères tel que défini précédemment.

Des modes de réalisation particuliers de ce procédé de traitement cosmétique résultent également de la description précédente. Le procédé de traitement cosmétique de l'invention peut être, notamment, mis en oeuvre en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

D'autres avantages et caractéristiques de l'invention apparaîtront mieux à la lecture des exemples donnés à titre illustratif et non limitatif.

**Exemple 1** : Préparation d'un l'hydrolysat de protéines fermentées de végétaux appartenant au genre des crucifères.

Dans une première étape, 1 kg des graines de colza sont délipidées par l'action d'un solvant organique : l'hexane. Après séchage du produit, on obtient un résidu enrichi en protéines (le tourteau). Une étape de fermentation est ensuite réalisée.

Le milieu de culture de la fermentation est constitué :

- d'un tourteau de colza à une concentration de 18g/l,
- de glucose à une concentration de 20g/l,
- de chlorure de sodium à une concentration de 4g/l,
- de $K_2HPO_4$ à une concentration de 2g/l.

Ce milieu est à un pH de 6,5. Il est ensemencé par des levures du genre Rhysopus. La culture s'effectue dans un fermenteur, sous agitation lente (30 tours/minute), à 30°C, durant 24 heures. Le mélange est ensuite mis à l'autoclave durant vingt minutes à 120°C puis il est hydrolysé, par ajout d'une enzyme : la papaïne, à 60°C pendant 4 heures sous agitation. Ce mélange est ensuite chauffé à 80 °C, centrifugé et filtré jusqu'à l'obtention d'une solution limpide. Il est ensuite concentré sous vide, puis filtré de nouveau sur des filtres-plaques puis sur cartouche stérilisante.

On obtient alors une hydrolysat de couleur brune titrant à 30g/l de composés de nature protéique. C'est-à-dire on obtient un hydrolysat contenant une quantité de composés de nature protéique représentant environ 65 % du poids total de la matière sèche. Cet hydrolysat est remis en solution dans du dipropylène glycol.

On peut, cependant, effectuer une étape d'extraction d'une fraction protéique à partir du tourteau au préalable de la fermentation ; cette fraction protéique servira ensuite de substrat à la fermentation. Cette extraction est réalisée à l'aide d'une solution aqueuse, basique (à pH 11) et à chaud (50°C), sous agitation constante durant une heure. Le milieu d'extraction est ensuite amené vers un pH de 3 ou 4, par une solution acide, préférentiellement par un acide minéral (acide chlorhydrique ou sulfurique). Un précipité de nature protéique est formé, il est recueilli par centrifugation puis par filtration. Ce mélange est remis en suspension afin de servir de substrat à la fermentation.

**Exemple 2** : Mise en évidence de l'effet dépigmentant de l'extrait de l'exemple 1 par des tests *ex vivo.*

L'activité dépigmentante de l'hydrolysat selon l'exemple 1 a été mise en évidence sur un échantillon de peau.

Des biopsies de 6 mm de diamètre sont prélevées sur des échantillons de peau humaine. Ces biopsies sont maintenues en survie *ex vivo* par culture en présence d'un milieu spécifique (DMEM 1 g/L, HAMF12, SVF et antibiotiques) sur des inserts déposés dans des plaques 6 puits. Les biopsies sont alors traitées avec l'actif à la concentration de 1 % suivant différentes conditions. Des contrôles, c'est-à-dire sans l'application d'actif, sont aussi réalisés pour chacune des conditions.

- Condition A : l'actif a été appliqué durant 5 jours, à raison de 2 applications par jour.
- Condition B : une irradiation de la peau à 100 mJ/cm$^2$ est réalisée, l'actif est ensuite appliqué 2 fois sur 24 heures.
- Condition C : un pré-traitement avec l'actif est réalisé pendant 24 heures (2 applications), suivi de 4 jours de culture sans application, puis une irradiation à 100 mJ/cm$^2$ est réalisée, l'actif est ensuite appliqué 2 fois sur 24 heures.
- Condition D : un pré-traitement avec l'actif est réalisé pendant 24 heures (2 applications), suivi d'une irradiation à 100 mJ/cm$^2$, l'actif est ensuite appliqué pendant 2 jours (2 applications).
- Condition E : un pré-traitement avec l'actif est réalisé pendant 24 heures (2 applications), suivi d'une irradiation à 100 mJ/cm$^2$, l'actif est ensuite appliqué pendant 5 jours (2 applications).

Une évaluation quantitative de la quantité de mélanine présente dans l'épiderme des échantillons de peau est réalisée histologiquement, au microscope optique, après une coloration suivant la méthode de Fontana-Masson.

Les biopsies de peau sont incluses dans la paraffine et des coupes histologiques de 4 $\mu$m d'épaisseur sont effectuées. Ces coupes sont alors colorées par la technique de Fontana-Masson : les lames sont déparaffinées, hydratées puis traitées avec une solution d'ammoniaque d'argent. Après un passage de deux minutes au micro-onde, les lames sont rincées, traitées avec du sodium thiosulfate, rincées à nouveau et contre-colorées avec de l'hématoxyline avant d'être déshydratées et montées sous lamelles permettant ainsi la visualisation au microscope optique de la mélanine présente dans l'épiderme.

La visualisation, au microscope optique, de la quantité de mélanine a permis de dénombrer trois types de pigmentation de la peau :

(+) : Peau fortement pigmentée, c'est-à-dire présentant une charge importante en mélanine (dépôt homogène).
(-) : Peau moyennement pigmentée, c'est-à-dire présentant une charge modérée en mélanine (grains de mélanines épars, dépôt non homogène).
(- -) : Peau faiblement pigmentée ou non pigmentée.

Les résultats sont rassemblés dans le tableau ci-dessous :

| Conditions : | Sans actif | Avec actif |
|---|---|---|
| A | - | -- |
| B | + | - |
| C | + | -- |
| D | + | - |
| E | + | -- |

Ces résultats nous permettent de conclure qu'en l'absence d'une irradiation par les uvb (condition A), l'actif diminue le taux de mélanine par comparaison avec l'échantillon de peau non traitée. Par ailleurs, la synthèse de mélanine, induite par les irradiations aux UVB, est atténuée lorsque l'actif est appliqué pendant 2 à 5 jours après irradiation. Cet effet est encore plus marqué lorsque les échantillons sont pré-traités avec l'actif avant l'irradiation. Ainsi, l'actif selon l'invention permet de diminuer significativement la pigmentation de la peau et permet, vraisemblablement, d'obtenir une inhibition de la synthèse de mélanine.

**Exemple 3** : Mise en évidence de l'effet dépigmentant de l'extrait de l'exemple 1 par une étude clinique (tests *in vivo*).

1. Principe du test

Un test *in vivo* a été effectué sur des volontaires de façon à mettre en évidence l'effet dépigmentant de l'hydrolysat selon l'exemple 1. Ce test a été réalisé par l'étude de l'index mélanique de la surface cutanée ainsi que par une analyse des zones de peau traitées avec l'actif

2. Modèle expérimental

Une étude clinique a été effectuée sur un groupe de 15 volontaires âgés de 45 à 76 ans. Cette étude a été réalisée en double aveugle contre placebo, les volontaires présentant une pigmentation non homogène de la peau (c'est-à-dire des taches pigmentaires) due à l'âge et/ou à une exposition aux UV. Les volontaires ont appliqué l'actif, formulé à 3 % dans une composition, sur une zone délimitée des avant-bras, dosé à 2 mg/cm$^2$, une autre zone recevant le placebo. Cette application a été réalisée deux fois par jours durant 4 semaines.
L'effet dépigmentant a été mesuré par une quantification de l'index mélanique réalisée à l'aide d'un instrument spécifique : le Mexameter MX18 (Courage & Khazaka). L'étude a comporté plusieurs visites de contrôle : une visite avant le début du traitement (T0), et une visite chaque semaine pendant 4 semaines. Des photos ont été prises à T0 et T4 à l'aide d'un appareil photo numérique (Minolta dimage 7i). L'évolution de la pigmentation cutanée a été évaluée après observation clinique ainsi que par la quantification de l'index mélanique.

3. Résultats

L'évolution de la pigmentation a été mesurée par la quantification de l'index mélanique de la peau, ainsi que par le traitement statistique des données. Les résultats sont présentés dans le tableau ci-dessous.
L'analyse statistique a été réalisée en utilisant le test non paramétrique de Wilcoxon pour les séries appariées, pour chaque sujet. Le résultat est obtenu selon le calcul suivant :

$$\text{Score} = \text{Diff.}_{A/Un \text{ ou } Pl} = T8_{A/Un \text{ ou } Pl} - T0_{A/Un \text{ ou } Pl}$$

A = Actif ; Un = Non traité ; Pl = Placebo

Région de rejet : dans la mesure où le sens de la différence est prédictible, la région de rejet sera unilatérale. Le niveau de significativité est $\alpha \leq 5$ %.

| | Index de mélanine | | % diminution | P |
|---|---|---|---|---|
| | T0 | T4 | | |
| **Actif** | 246.400 | 211.467 | - 6.595 | 0.00235*** |
| **Placebo** | 237.333 | 236.267 | -0.449 | 0.4875ns |
| (***) : très significatif (ns) : non significatif | | | | |

Ces résultats démontrent que l'application de l'actif selon l'invention réduit considérablement le taux de mélanine présent dans la peau. Cette diminution est notamment observée au niveau des régions hyperpigmentées et après 4 semaines d'application de l'actif, cette diminution étant statistiquement signifiante. Ces résultats ont, par ailleurs, été confirmés après examen clinique : 67 % des volontaires ont présenté une diminution de la pigmentation de la région traitée avec l'actif par rapport à la région traitée avec le placebo. Par ailleurs, il a été noté, chez 86 % des volontaires, une diminution apparente des taches pigmentaires.

En conclusion, il est constaté que l'hydrolysat selon l'invention, formulé à 3 % dans un gel, possède un effet dépigmentant, c'est-à-dire un effet éclaircissant de la peau ainsi qu'une action véritable dans la lutte contre les taches pigmentaires.

**Exemple 4 :** Mise en évidence de l'effet de l'extrait de l'exemple 1 sur la synthèse de mélanine par les mélanocytes en culture.

1. Détermination du taux de mélanine

Le principe de ce test repose sur un dosage de la mélanine par une méthode spectrométrique.

Des boites « diamètre 60 » sont ensemencées avec $1.10^5$ cellules, puis mises en culture durant 24 heures. Ces cellules sont, par la suite, traitées avec l'extrait selon l'invention, en solution à différents pourcentages : 1 %, 3 % et 7% et durant 24 heures.

Les cellules sont ensuite récupérées par trypsination. Une moitié de ces cellules est alors utilisée pour le dosage de la mélanine et l'autre moitié pour la détermination du contenu protéique (par la technique de Pierce). Afin de réaliser le dosage de la mélanine, les cellules sont solubilisées dans 1 mL de NaOH-1N / 10 %-DMSO pendant 2 heures à 80°C, puis centrifugées durant 10 minutes à 10000 g. L'absorbance du surnageant est alors lue à 470 nm puis comparée avec la courbe étalon de la mélanine. Cette courbe étalon est préparée à l'aide de la mélanine synthétique SIGMA ayant des concentrations comprises entre 0,05 et 100 $\mu$g/mL et avec une concentration finale en NaOH à 0,2M.

Les résultats sont présentés dans le tableau ci-dessous. Il représent la quantité de mélanine synthétisée, exprimée en pg/$\mu$g de protéines, en fonction des différentes conditions d'études.

| Conditions d'études : | Contrôles | Avec extrait à 1 % | Avec extrait à 3 % | Avec extrait à 7 % |
|---|---|---|---|---|
| Qté mélanine (pg/$\mu$g de protéines). | 171 | 126 | 110 | 107 |

Ces résultats démontrent que l'extrait selon l'invention permet de faire diminuer significativement la quantité de mélanine présente dans les mélanocytes, cet extrait agissant de manière dose-dépendante.

2. Détermination de l'activité de la tyrosinase

La tyrosinase est une enzyme-clé dans le mécanisme de formation de la mélanine. La mesure de son activité permet de déterminer la capacité de l'actif selon l'invention à inhiber le mécanisme de formation de celle-ci. Le principe de ce test repose sur une mesure du taux d'oxydation d'un substrat : la L-DOPA.

Les cellules sont mises en cultures dans des plaques 6 puits (avec $1.10^5$ cellules) durant 24 heures. Elles sont, par la suite, traitées avec l'extrait selon l'invention mis en solution à 3 % durant 24 heures, 48 heures ou 72 heures.

Les cellules sont récupérées par trypsination, lavées 3 fois avec du PBS froid puis centrifugées durant 5 minutes à 10000 g. Les cellules sont lysées avec 300$\mu$L de tampon sodium phosphate (0,1 M, pH 7) contenant 1 % de triton X-100 + 0,1 mM PMSF. Après une incubation de 30 minutes, l'extrait cellulaire est centrifugé à 10000 g pendant 10 minutes à 4°C, le surnageant est alors récupéré. Le contenu protéique de chaque extrait est déterminé par la technique de Pierce. La L-DOPA est préparée à 2 mg/mL (10 mM) dans un tampon phosphate (0,1 M ; pH7) ; un volume de 10µL de chaque extrait est placé dans une plaque 96 puits et la mesure de l'activité enzymatique est démarée en ajoutant 100µL de la solution L-DOPA à 37°C, les puits « contôles », contenant 100 µL de tampon de lyse, sont réalisés. La formation du dopachrome est suivie par une mesure de l'absorbance à 405 nm, toutes les 10 minutes, durant 1 heure, à 37°C. Une courbe d'absorbance est alors établie pour chaque condition.

L'activité finale de la tyrosinase est présentée en DO/min/g de protéines suivant les différentes conditions d'étude réalisées. Les résultats obtenus sont présentés dans le tableau ci-dessous.

| Activité de la tyrosinase (DO/min/g de prot.) | Traitement durant 24 h. | Traitement durant 48 h. | Traitement durant 72 h. |
|---|---|---|---|
| Cellules non traitées | 130 | 83 | 54 |
| Cellules traitées | 124 | 74 | 46 |

Ces résultats nous permettent de déduire que l'extrait selon l'invention inhibe de manière particulièrement efficace l'activité de la tyrosinase, enzyme-clé dans le mécanisme de formation de la mélanine

**Exemple 5 :** Préparation de compositions.

Les quantités indiquées sont données en pourcentage de poids.

**1** - <u>Crème éclat du teint avec protection solaire :</u>

| Noms commerciaux | Noms INCI | % massique |
|---|---|---|
| **PHASE A** | | |
| Montanov 68 | Cetearyl Alcohol (and) Cetearyl Glucoside | 5.00 |
| Isopropyl Palmitate | Isopropyl Palmitate | 7.00 |
| Waglinol 250 | Cetearyl Ethylhexanoate | 3.00 |
| Dow Corning 200 | Dimethicone Polydimethylsiloxane | 0.50 |
| Parsol MCX | Ethylhexyl Methoxycinnamate | 3.00 |
| Parsol 1789 | Butyl Methoxydibenzoylmethane | 1.00 |
| Phenonip | Phenoxyethanol (and) Methylparaben (and) Ethylparaben (and) Butylparaben (and) Propylparaben (and) Isobutylparaben | 0.50 |
| Cegesoft PS6 | Vegetable Oil | 2.00 |
| Huile de Jojoba | Simmondsia Chinensis (Jojoba) Seed Oil | 5.00 |
| **PHASE B** | | |
| Eau Demineralisee | Aqua (Water) | qsp |
| Glycerine | Glycerin | 3.00 |
| Glucam E10 | Methyl Gluceth-10 | 0.50 |
| EDTA Tetrasodium | EDTA | 0.20 |
| **PHASE C** | | |
| Sepigel 305 | Polyacrylamide (and) C13-14 Isoparaffin (and) Laureth-7 | 0.35 |
| Jus de Citron | Citrus medica Limonum (Lemon) Fruit Extract | 0.23 |

(suite)

| PHASE D | | |
|---|---|---|
| Extrait selon l'exemple 1 | | 1.50 |
| Compo parfumante | Parfum (Fragrance) | qsp |
| Colorant | Dye | qsp |

Les constituants de la phase A et de la phase B sont chauffés à une température de 70°C, puis la phase A est émulsionnée dans la phase B. Après émulsion, le Sepigel est incorporé suivi du jus de citron. La phase D est, par la suite, additionnée lorsque la température se situe en dessous de 40°C.

**2** - Gel mains anti-tâches :

| Noms commerciaux | Noms INCI | % massique |
|---|---|---|
| **PHASE A** | | |
| Eau déminéralisée | Aqua (water) | qsp |
| Glycerine | Glycerin | 16.00 |
| Butylene Glycol | Butylene Glycol | 6.00 |
| Germall 115 | Imidazolidinyl Urea | 0.30 |
| Allantoïne | Allantoïn | 0.20 |
| Sepigel 305 | Polyacrylamide (and) C13-14 isoparaffin (and) Laureth-7 | 3.00 |
| **PHASE B** | | |
| Dow Coming 345 Fluid | Cyclomethicone | 5.00 |
| Phenonip | Phenoxyethanol (and) Methylparaben (and) Ethylparaben (and) Butylparaben (and) Propylparaben (and) Isobutylparaben | 0.50 |
| **PHASE C** | | |
| Dry Flo Pure | Aluminium Starch Octenylsuccinate | 4.00 |
| Extrait selon l'exemple 1 | | 1.00 |
| Parfum | Parfum (Fragrance) | qsp |
| Colorant | Dye | qsp |

La phase A est préparée en mélangeant les différentes matières premières la constituant, à 30°C, sous agitation. La phase B est jointe à la phase A en mélangeant avec une agitation à hélice. La phase C est saupoudrée dans le vortex.

**3** - Sérum dépigmentant :

| Noms commerciaux | Noms INCI | % massique |
|---|---|---|
| **PHASE A** | | |
| Natrosol 250 HR | Hydroxyethylcellulose | 0.60 |
| Propylene Glycol | Propylene Glycol | 5.00 |
| Eau Déminéralisée | Aqua (Water) | qsp |

(suite)

| PHASE B | | |
|---|---|---|
| EDTA Tetrasodium Salt | EDTA | 0.05 |
| Ethanol 96% | Alcohol | 5.00 |
| **PHASE C** | | |
| Eau déminéralisée | Aqua (Water) | qsp |
| C-Mate | Magnesium Ascorbyl Phosphate | 0.50 |
| **PHASE D** | | |
| DL-Lactic Acid | Lactic Acid | 0.10 |
| Extrait selon l'exemple 1 | | 3.00 |
| Composition parfumante | Parfum (Fragrance) | qsp |
| Colorant | Dye | qsp |

La phase A est préparée à une température voisine de 30°C, on laisse gonfler le gel ainsi obtenu durant ½ heure. La phase B est ensuite incorporée à froid. La phase C est préparée sous agitation à froid et elle est incorporée au gel. La phase D est ensuite additionnée sous agitation.

**Revendications**

1. Utilisation d'une quantité efficace d'un hydrolysat de protéines de plantes, appartenant à la famille des crucifères, comme principe actif blanchissant et/ou dépigmentant, pour la préparation d'une composition thérapeutique pour blanchir et/ou dépigmenter.

2. Utilisation non-thérapeutique d'une quantité efficace d'un hydrolysat de protéines de plantes, appartenant à la famille des crucifères, comme principe actif blanchissant et/ou dépigmentant pour blanchir et/ou dépigmenter.

3. Utilisation selon l'une des revendications 1 ou 2, **caractérisée en ce que** les protéines sont des protéines fermentées de plantes appartenant à la famille des crucifères.

4. Utilisation selon l'une des revendications précédentes, **caractérisée en ce que** la plante de la famille des crucifères utilisée est le colza (Brassica napus).

5. Utilisation selon l'une des revendications précédentes, **caractérisée en ce que** l'hydrolysat est obtenu à partir des graines de colza.

6. Utilisation selon l'une des revendications précédentes, **caractérisée en ce que**, dans l'étape de fermentation, le milieu de culture est supplémenté en sucres, en acides aminés et en sel minéraux.

7. Utilisation selon l'une quelconque des revendications précédentes, **caractérisée en ce que** l'hydrolysat contient une quantité de composés de nature protéique représentant entre 30 et 90 % du poids total de la matière sèche.

8. Utilisation selon l'une quelconque des revendications précédentes, **caractérisée en ce que** l'hydrolysat contient une quantité de composés de nature protéique représentant entre 50 et 80 % du poids total de la matière sèche.

9. Utilisation selon l'une quelconque des revendications 1 à 8, l'hydrolysat ou la composition étant destinés à dépigmenter et/ou blanchir et/ou éclaircir la peau, les poils et/ou les cheveux.

10. Utilisation selon l'une quelconque des revendications 1 à 8, l'hydrolysat ou la composition étant destinés à enlever ou diminuer les taches pigmentaires de la peau.

11. Utilisation selon l'une quelconque des revendications 1 à 8, l'hydrolysat ou la composition étant destinés à inhiber et/ou diminuer l'activité de la tyrosinase, et/ou afin d'inhiber et/ou de diminuer la synthèse de mélanine des cellules de la peau.

12. Utilisation selon l'une quelconque des revendications précédentes, **caractérisée en ce que** la composition se présente sous la forme d'une composition adaptée à l'administration par voie topique cutanée comprenant un milieu cosmétiquement ou pharmaceutiquement acceptable.

13. Utilisation selon l'une quelconque des revendications précédentes, **caractérisée en ce que** l'hydrolysat de protéine est présent en une quantité représentant de 0,0001 % à 20 % du poids total de la composition, et préférentiellement en une quantité représentant de 0,01 % à 10 % du poids total de la composition.

14. Utilisation selon l'une quelconque des revendications précédentes **caractérisée en ce que** l'hydrolysat est préalablement solubilisé dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés ou tout mélange de ces solvants.

15. Utilisation selon l'une quelconque des revendications précédentes, **caractérisée en ce que** l'hydrolysat est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

16. Utilisation selon l'une quelconque des revendications précédentes, **caractérisée en ce que** la composition se présente sous forme d'une solution aqueuse, hydroalcoolique ou huileuse ou sous la forme d'une émulsion huile-dans-eau, eau-dans-huile ou émulsions multiples ou sous forme de crèmes, de suspensions ou encore de poudres ; ces compositions pouvant être plus ou moins fluides ou solides et avoir l'aspect d'une crème, d'une lotion, d'un lait, d'un sérum, d'une pommade, d'un gel, d'une pâte, d'une mousse ou d'un stick.

17. Procédé de traitement cosmétique de la peau destiné à éclaircir la peau et/ou les poils; et/ou à traiter les désordres de la pigmentation de la peau, **caractérisé par le fait que** l'on applique sur la peau, les poils ou les cheveux une composition cosmétique contenant, dans un milieu cosmétiquement acceptable, au moins un hydrolysat de protéines de plantes appartenant à la famille des crucifères.

**Claims**

1. Use of an effective quantity of a protein hydrolysate from plants, belonging to the Cruciferae family, as a whitening and/or depigmenting active ingredient, for the preparation of a therapeutic composition for whitening and/or depigmentation.

2. Non-therapeutic use of an effective quantity of a protein hydrolysate from plants, belonging to the Cruciferae family, as a whitening and/or depigmenting active ingredient for whitening and/or depigmentation.

3. Use according to any of claims 1 or 2, **characterised in that** the proteins are fermented proteins from plants belonging to the Cruciferae family.

4. Use according to any of the above claims, **characterised in that** the plant from the Cruciferae family used is rapeseed (Brassica napus).

5. Use according to any of the above claims, **characterised in that** the hydrolysate is obtained from rapeseeds.

6. Use according to any of the above claims, **characterised in that**, in the fermentation step, the culture medium is supplemented with sugars, amino acids and minerals.

7. Use according to any of the above claims, **characterised in that** the hydrolysate contains a quantity of protein compounds representing between 30 and 90% of the total weight of the dry substance.

8. Use according to any of the above claims, **characterised in that** the hydrolysate contains a quantity of protein compounds representing between 50 and 80% of the total weight of the dry substance.

9. Use according to any of claims 1 to 8, the hydrolysate or the composition being intended to depigment and/or whiten and/or lighten the skin, body hair and/or hair.

10. Use according to any of claims 1 to 8, the hydrolysate or the composition being intended to remove or reduce pigmentary skin blemishes.

11. Use according to any of claims 1 to 8, the hydrolysate or composition being intended to inhibit and/or reduce tyrosinase activity, and/or to inhibit and/or reduce skin cell melanin synthesis.

12. Use according to any of the above claims, **characterised in that** the composition is in the form of a suitable formulation for topical administration on the skin comprising a cosmetically or pharmaceutically acceptable medium.

13. Use according to any of the above claims, **characterised in that** the protein hydrolysate is present in a quantity representing from 0.0001% to 20% of the total weight of the composition, and preferentially in a quantity representing from 0.01% to 10% of the total weight of the composition.

14. Use according to any of the above claims **characterised in that** the hydrolysate is previously solubilised in one or more cosmetically or pharmaceutically acceptable solvents such as water, ethanol, propanol or isopropanol, propylene glycol, butylene glycol, dipropylene glycol, ethoxylated or propoxylated diglycols or any mixture of these solvents.

15. Use according to any of the above claims, **characterised in that** the hydrolysate is previously solubilised in a cosmetic or pharmaceutical vector such as liposomes or adsorbed on powdery organic polymers, mineral substrates such as talcs and bentonites, and more generally solubilised in, or fixed on, any cosmetically or pharmaceutically acceptable vector.

16. Use according to any of the above claims, **characterised in that** the composition is presented in the form of an aqueous, hydroalcoholic or oily solution or in the form of an oil-in-water, water-in-oil emulsion or multiple emulsions or in the form of creams, suspensions or powders; these compositions possibly being more or less fluid or solid and having the appearance of a cream, lotion, milk, serum, ointment, gel, paste, foam or stick.

17. Cosmetic skin treatment method intended to lighten the skin and/or body hair; and/or to treat skin pigmentation disorders, **characterised in that** a cosmetic formulation containing, in a cosmetically acceptable medium, at least one protein hydrolysate from plants belonging to the Cruciferae family is applied on the skin, body hair or hair.

**Patentansprüche**

1. Verwendung einer wirksamen Menge eines Hydrolysats von Proteinen von Pflanzen, die zur Familie der Kreuzblütler gehören, als bleichendes und / oder depigmentierendes Wirkprinzip zur Zubereitung einer therapeutischen Zusammensetzung zum Bleichen und / oder Depigmentieren.

2. Nicht-therapeutische Verwendung einer wirksamen Menge eines Hydrolysats von Proteinen von Pflanzen, die zur Familie der Kreuzblütler gehören, als bleichendes und / oder depigmentierendes Wirkprinzip zum Bleichen und / oder Depigmentieren.

3. Verwendung nach einem der Ansprüche 1 oder 2, **dadurch gekennzeichnet, dass** die Proteine fermentierte Proteine von Pflanzen sind, die zur Familie der Kreuzblütler gehören.

4. Verwendung nach einem der vorangehenden Ansprüche, **dadurch gekennzeichnet, dass** die verwendete Pflanze aus der Familie der Kreuzblütler der Raps (Brassica napus) ist.

5. Verwendung nach einem der vorangehenden Ansprüche, **dadurch gekennzeichnet, dass** das Hydrolysat aus Rapssaat gewonnen wird.

6. Verwendung nach einem der vorangehenden Ansprüche, **dadurch gekennzeichnet, dass** das Kulturmilieu beim Schritt der Fermentation mit Zuckern, Aminosäuren und Mineralsalzen supplementiert wird.

**7.** Verwendung nach einem der vorangehenden Ansprüche, **dadurch gekennzeichnet, dass** das Hydrolysat eine Menge proteinartiger Verbindungen enthält, die zwischen 30 und 90% des Gesamtgewichts der Trockenmasse darstellen.

**8.** Verwendung nach einem der vorangehenden Ansprüche, **dadurch gekennzeichnet, dass** das Hydrolysat eine Menge proteinartiger Verbindungen enthält, die zwischen 50 und 80% des Gesamtgewichts der Trockenmasse darstellen.

**9.** Verwendung nach einem der Ansprüche 1 bis 8, wobei das Hydrolysat oder die Zusammensetzung dazu bestimmt sind, die Haut, die Körperbehaarung und / oder das Haar zu depigmentieren und / oder zu bleichen und /oder aufzuhellen.

**10.** Verwendung nach einem der Ansprüche 1 bis 8, wobei das Hydrolysat oder die Zusammensetzung dazu bestimmt sind, Pigmentflecke der Haut zu entfernen oder zu mildern.

**11.** Verwendung nach einem der Ansprüche 1 bis 8, wobei das Hydrolysat oder die Zusammensetzung dazu bestimmt sind, die Aktivität der Tyrosinase zu inhibieren und / oder abzuschwächen und / oder um die Melaninsynthese der Hautzellen zu inhibieren und / oder abzuschwächen.

**12.** Verwendung nach einem der vorangehenden Ansprüche, **dadurch gekennzeichnet, dass** sich die Zusammensetzung in Form einer Zusammensetzung präsentiert, die zur Verabreichung auf topischem Weg über die Haut geeignet ist und ein kosmetisch oder pharmazeutisch akzeptables Milieu enthält.

**13.** Verwendung nach einem der vorangehenden Ansprüche, **dadurch gekennzeichnet, dass** das Proteinhydrolysat in einer Menge vorhanden ist, die 0,0001% bis 20% des Gesamtgewichts der Zusammensetzung darstellt, und vorzugsweise in einer Menge, die 0,01% bis 10% des Gesamtgewichts der Zusammensetzung darstellt.

**14.** Verwendung nach einem der vorangehenden Ansprüche, **dadurch gekennzeichnet, dass** das Hydrolysat zuvor in einem oder mehreren kosmetisch oder pharmazeutisch akzeptablen Lösungsmitteln wie Wasser, Ethanol, Propanol oder Isopropanol, Propylenglycol, Butylenglycol, Dipropylenglycol, ethoxylierten oder propoxylierten Diglycolen oder jedem Gemisch dieser Lösungsmittel gelöst wird.

**15.** Verwendung nach einem der vorangehenden Ansprüche, **dadurch gekennzeichnet, dass** das Hydrolysat zuvor in einem kosmetisch oder pharmazeutisch akzeptablen Vektor wie Liposome gelöst wird oder auf pulverförmigen organischen Polymeren, mineralischen Trägern wie Talke und Bentonite adsorbiert und allgemeiner in jedem kosmetisch oder pharmazeutisch akzeptablen Vektor gelöst oder auf ihm fixiert wird.

**16.** Verwendung nach einem der vorangehenden Ansprüche, **dadurch gekennzeichnet, dass** sich die Zusammensetzung in Form einer wässrigen, hydroalkoholischen oder öligen Lösung darstellt oder in Form einer Öl-in-Wasser-, Wasser-in-Öl-Emulsion oder multipler Emulsionen oder in Form von Cremes, Suspensionen oder auch Pulvern darstellt, wobei diese Zusammensetzungen mehr oder weniger flüssig oder fest sein und das Aussehen einer Creme, einer Lotion, einer Milch, eines Serums, einer Pomade, eines Gels, einer Paste, eines Schaums oder eines Sticks haben können.

**17.** Verfahren zur kosmetischen Behandlung der Haut, das dazu bestimmt ist, die Haut und / oder die Körperbehaarung aufzuhellen und / oder um Pigmentunregelmäßigkeiten der Haut zu behandeln, **dadurch gekennzeichnet, dass** man auf die Haut, die Körperbehaarung oder das Haar eine kosmetische Zusammensetzung aufträgt, die in einem kosmetisch akzeptablen Milieu mindestens ein Proteinhydrolysat von Pflanzen enthält, die zur Familie der Kreuzblütler gehören.

**RÉFÉRENCES CITÉES DANS LA DESCRIPTION**

**Documents brevets cités dans la description**

- GB 1349955 A
- EP 98401360 A
- FR 2802417